# EUROPÄISCHE PATENTSCHRIFT

(11) **EP 1 144 386 B1**
(45) Veröffentlichungstag und Bekanntmachung des Hinweises auf die Patenterteilung: **22.10.2003**
(21) Anmeldenummer: 00901510.8
(22) Anmeldetag: 07.01.2000
(51) Int. Cl.: C07D 235/18, A61K 31/4184, A61P 9/12, A61P 9/10

(54) **POLYMORPHE VON TELMISARTAN, VERFAHREN ZU DEREN HERSTELLUNG UND DEREN VERWENDUNG ZUR HERSTELLUNG EINES ARZNEIMITTELS**
TELMISARTAN POLYMORPHS, METHODS FOR PRODUCING SAME AND THEIR USE IN THE PREPARATION OF A MEDICAMENT
POLYMORPHES DE TELMISARTANE, PROCEDES PERMETTANT DE LES PREPARER ET LEUR UTILISATION POUR PREPARER UN MEDICAMENT

(30) Priorität: 19.01.1999 DE 19901921
(43) Veröffentlichungstag der Anmeldung: 17.10.2001
(73) Patentinhaber: Boehringer Ingelheim Pharma GmbH & Co.KG, 55218 Ingelheim am Rhein (DE)
(72) Erfinder: SCHNEIDER, Heinrich, D-55218 Ingelheim am Rhein (DE)
(86) Internationale Anmeldenummer: EP0000065
(87) Internationale Veröffentlichungsnummer: WO00043370

(56) Entgegenhaltungen:
- EP-A- 0 502 314
- RIES U J ET AL: "6-Substituted benzimidazoles as new nonpeptide angiotensin II receptor antagonists: synthesis, biological activity, and structure-activity relationships" J. MED. CHEM. , Bd. 36, Nr. 25, 1993, Seiten 4040-4051, XP002135628

## Beschreibung

Die Erfindung betrifft Polymorphe von 4'-[2-n-Propyl-4-methyl-6-(1-methylbenzimidazol-2-yl)benzimidazol-1-ylmethyl]biphenyl-2-carbonsäure (INN: Telmisartan), insbesondere die polymorphe Form B, Gemische der Polymorphen, Verfahren zur Herstellung von Form B enthaltendem Telmisartan sowie dessen Verwendung zur Herstellung eines Arnzeimittels.

### Hintergrund der Erfindung

Die Verbindung Telmisarten ist aus dem Europäischen Patent

EP 502 314 B1 sowie auch aus Ries et al. (J. Med. Chem **36**(25), 4040-4051 (1993)) bekannt und weist die folgende chemische Struktur auf:

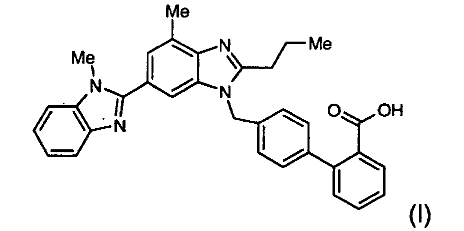

Telmisartan, sowie dessen physiologisch verträgliche Salze, besitzen wertvolle pharmakologische Eigenschaften. Telmisartan stellt einen Angiotensin-Antagonisten, insbesondere einen Angiotensin-II-Antagonisten dar, der aufgrund seiner pharmakologischen Eigenschaften bespielsweise zur Behandlung der Hypertonie und Herzinsuffizienz, zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Neuropathie, des Glaukoms, von gastrointestinalen Erkrankungen sowie von Blasenerkrankungen Verwendung finden kann. Weitere mögliche Therapiegebiete sind der EP 502314 B1 zu entnehmen, auf die an dieser Stelle inhaltlich Bezug genommen wird.

Im Zuge der Telmisartan-Synthese wird als abschließender Synthesschritt die Verseifung des tert-Butylesters (II) gemäß Schema 1 durchgeführt.

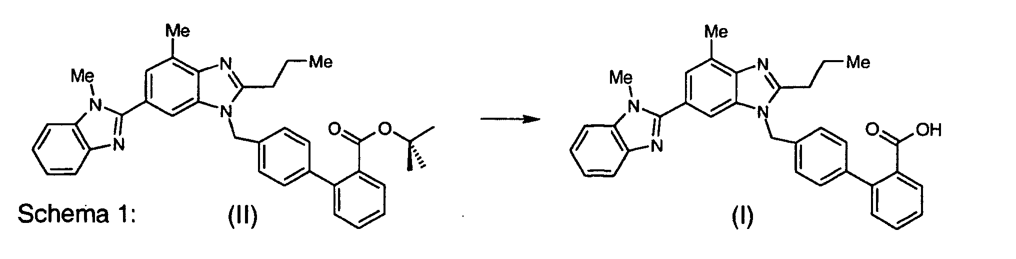

Die entsprechende, im Labormaßstab durchführbare experimentelle Arbeitsvorschrift ist der EP 502314 B1 zu entnehmen. Allerdings war eine Übertragung des bereits bekannten Syntheseverfahrens in einen großtechnischen Herstellungsprozess überraschenderweise nicht problemlos durchführbar. Das gemäß Schema 1 großtechnisch synthetisierte Telmisartan fällt nach Aufarbeitung in Form eines Produktes an, welches zur abschließenden Reinigung einem weiteren Kristallisationschritt unterworfen werden muß. Bei besagtem, zwingend erforderlichen Kristallisationsschritt führte die Morphologie des auskristallisierenden Endprodukts zu unvorhergesehenen Schwierigkeiten.

Das in Form langer Nadeln als Feststoff ausfallende Produkt kann nur schwer filtriert, gewaschen und isoliert werden, zeichnet sich ferner aufgrund des Einschlusses von Lösungsmittel durch eine sehr lange Trocknungszeit aus und bildet beim Trocknungsprozess große, sehr harte Brocken. Eine Zerkleinerung dieser Brocken führt zu einem trockenen Pulver welches eine hohe Tendenz zur elekrostatischen Aufladung zeigt und praktisch nicht rieselfähig ist.

Obige, nachteilige Eigenschaften eines Produkts erweisen sich bei der großtechnischen Herstellung einer Verbindung stets als aüßerst hinderlich, da sie deren reproduzierbare Bereitstellung in größerer Menge und hoher Reinheit nur unter großen Schwierigkeiten oder zusätzlichem, hohem technischen Aufwand zulassen.

Es ist daher Aufgabe der vorliegenden Erfindung, Telmisartan in einer Form bereitzustellen, die die großtechnisch durchführbare Synthese, Aufarbeitung, Reinigung und Isolierung von Telmisartan erlaubt, bei der die vorstehend genannten Nachteile überwunden werden.

### Detaillierte Beschreibung der Erfindung

Überraschenderweise wurde gefunden, daß Telmisartan als Feststoff in unterschiedlichen Kristallmodifikationen vorliegen kann. Je nach Art des Kristallisationsprozesses ist es in zwei unterschiedliche polymorphe Formen A und B überführbar.

Beim Polymorph A handelt es sich um die gemäß dem Stand der Technik zugängliche Form des Telmisartans, die die vorstehend genannten Schwierigkeiten bei der großtechnischen Herstellung bzw. Reinigung, Isolierung und Trocknung des Produktes verursacht.

Die überraschend gefundene polymorphe Form B des Telmisartans zeigt dagegen nahezu keine Neigung zur elektrostatischen Aufladung, läßt sich hervorragend absaugen, zentrifugieren, waschen und trocknen und ist auch ohne Zerkleinerung rieselfähig.

Zur Herstellung der polymorphen Form B des Telmisartans wird erfindungsgemäß wie folgt vorgegangen.

In einem entsprechend dimensionierten Rührwerksapparat wird Telmisartan-Rohprodukt (kristallisiert beispielsweise aus Dimethylformamid, Dimethylacetamid oder ähnlichem) gegebenenfalls mit 1-5Gew.-%, bevorzugt mit 3Gew.-% Aktivkohle in einem Lösemittelgemisch bestehend aus Wasser, Ameisensäure und einem geeigneten organischen Lösemittel aufgenommen und anschließend bei erhöhter Temperatur, bevorzugt bei einer Temperatur von 50-90°C, besonders bevorzugt bei 60-80°C gelöst.

Erfindungswesentlich ist der Einsatz des Lösemittelgemisches Ameisensäure/Wasser mit einem organischen Lösemittel welches erfindungsgemäß die folgenden Kriterien erfüllen muß. Es muß zur Bildung einer Lösung mit dem Gemisch Ameisensäure/Wasser befähigt sein Es muß chemisch weitgehend inert gegenüber dem Gemisch Ameisensäure/Wasser sein und es muß vom Gemisch Ameisensäure/Wasser destillativ abtrennbar sein. Verwendung finden können organische Carbonsäureester, Ketone oder Ether. Beispielhaft seien genannt Aceton, Methylethylketon, Methylacetat, Ethylacetat, Ethylformiat, Ethylenglykoldimethylether oder Tetrahydrofuran. Erfindungsgemäß bevorzugt sind Aceton, Methylethylketon, Methylacetat, Ethylacetat, THF, besonders bevorzugt ist Ethylacetat.

Das Lösemittelgemisch sollte erfindungsgemäß pro Mol Telmisartan zusammengesetzt sein aus 0.3-0.7 I Wasser, 10-15 Mol Ameisensäure und 0.3-0.9 l des organischen Lösemittels. Bevorzugt ist ein Verhältnis von 0.4-0.6 I Wasser, 11-13 Mol Ameisensäure und 0.4-0.7 I des organischen Lösemittels bezogen auf 1 Mol Telmisartan. Besonders bevorzugt ist ein Verhältnis von ca. 0.5 I Wasser, ca. 11.5-12 Mol Ameisensäure und ca. 0.5 l des organischen Lösemittels bezogen auf 1 Mol Telmisartan.

Erfindungsgemäß wird nach der eingangs genannten Erwärmung die erhaltene Lösung filtriert und mit einem Gemisch des vorstehend genannten organischen Lösemittels mit Ameisensäure nachgewaschen. Pro Mol Telmisartan kann die Waschlösung 0.3-1.0 Mol, bevorzugt 0.4-0.6 Mol, besonders bevorzugt ca. 0.5 Mol Ameisensäure enthalten. Die Menge der Waschlösung bestimmt sich naturgemäß aus der Menge des gelösten Telmisartans. Erfindungsgemäß werden pro Mol Telmisartan 0.1-0.4, bevorzugt 0.15-0.3, besonders bevorzugt 0.2 l des organischen Lösemittels eingebracht.

Nach Nachwaschen des Filtrationsrückstandes mit vorstehend beschriebener Waschlösung wird unter gleichzeitiger Wasserzudosierung das organische Lösemittel möglichst vollständig abdestilliert. Dabei wird die Temperatur in einem Bereich von 60-100°C, bevorzugt zwischen 70-100°C gehalten. Die insgesammt zudosierte Wassermenge entspricht im Wesentlichen der Gesamtmenge an abdestilliertem Lösemittel. Ein praktisch vollständiges Abdestillieren des organischen Lösemittels ist erfindungsgemäß erwünscht. Entsprechend wird die Destillation soweit geführt, daß auch Wasser, teilweise azeotrop, mit abdestilliert wird. Das abdestillierte organische Lösemittel kann, gegebenenfalls nach Abtrennung der Wasserphase, wieder in Folgeumsetzungen eingesetzt werden.

Zur Fällung des Telmisartan-polymorphen B wird anschließend auf einen Temperaturbereich von 15-60°C, bevorzugt auf 20-30°C abgekühlt, und mit einer Base gefällt. Die Menge an einzusetzender Base ist von der Menge der eingesetzten Ameisensäure abhängig. Bevorzugt werden 0-2 Mol weniger Base zugesetzt als Ameisensäure enthalten ist. Besonders bevorzugt werden 0.3-1.5 Mol weniger Base zugesetzt als Ameisensäure enthalten ist. Höchst bevorzugt werden 0.5-1 Mol weniger Base zugesetzt als Ameisensäure enthalten ist. Als Base kommen sowohl wässrige Lösungen des Kaliumhydroxids, Natriumhydroxids, Lithiumhydroxids oder Ammoniaks in Betracht. Verwendung finden können ferner geeignete organische Basen wie Triethylamin, Diisopropylethylamin oder auch DBU (Diazabicycloundecen). Besonders bevorzugt sind als Basen die vorstehend genannten wässrigen Lösungen des Kaliumhydroxids, Natriumhydroxids, Lithiumhydroxids oder Ammoniaks von denen den wässrigen Lösungen des Ammoniaks eine besondere Bedeutung zukommt.

Das ausgefallene Produkt wird abgeschleudert, mit Wasser gewaschen und üblicherweise im Vakuum bei 120-125°C getrocknet.

Ein direkt nach dem Schleudern genommenes und im Labor in dünner Schicht im Umlufttrockenschrank getrocknetes Muster zeigt typischerweise einen Gehalt von 95-99% der Kristallmodifikation B.

Nach dem Zentrifugieren beginnt sich das Produkt, abhängig von Temperatur, pH, Verweildauer und Wassergehalt, bis gegen Ende der Trocknung teilweise in Modifikation A umzuwandeln. Bei Betriebsansätzen werden deshalb nach dem Trocknen Verhältnisses von Form A zu Form B von bestenfalls ca. 10:90, aber auch Verhältnisse von 60:40 erhalten.

Allerdings garantiert auch ein derart niedriger Gehalt an Form B die bei der großtechnischen Herstellung erforderlichen positiven Eigenschaften des Produkts (z.B. geringe Tendenz zur elektrostatischen Aufladung, geringe Tendenz zur Verklumpung, Rieselfähigkeit etc.). Erfindungswesentlich beim vorstehend genannten Kristallisationsprozess ist, daß zunächst lediglich Form B mit ihrer charakteristischen makroskopischen Kristallform entsteht. Diese makroskopische Kristallform bleibt unter den Trocknungsbedingungen trotz partieller mikroskopischer Umformung in Form A weitgehend erhalten.

Weitere sehr vorteilhafte Aspekte der erfindungsgemäßen Vorgehensweise sind die hohe Raum-Zeit-Ausbeute beim vorliegenden Prozess sowie die hohe Ausbeute an Telmisartan-Reinprodukt, welches fast quantitativ isoliert werden kann.

Das nach dem aus dem Stand der Technik bekannten Herstellungsverfahren erhältliche Telmisartan der Form A unterscheidet sich von erfindungsgemäß zugänglichen Telmisartan, welches durch einen Gehalt an polymporpher Form B gekennzeichnet ist, in den bereits eingangs erwähnten, vorteilhaften Produkteigenschaften. Weitere Unterscheidungsmerkmale werden im Folgenden beschrieben.

Telmisartan der Form A kristallisiert in langen, feinen bzw. dünnen Nadeln, die filzartig aneinanderhaften. Die Kristallmodifikation des Telmisartans der Form B bildet sehr kompakte, würfel- bis kugelförmige Kristalle aus, die sand- oder kieselgelartiges Rieselverhalten aufweisen.

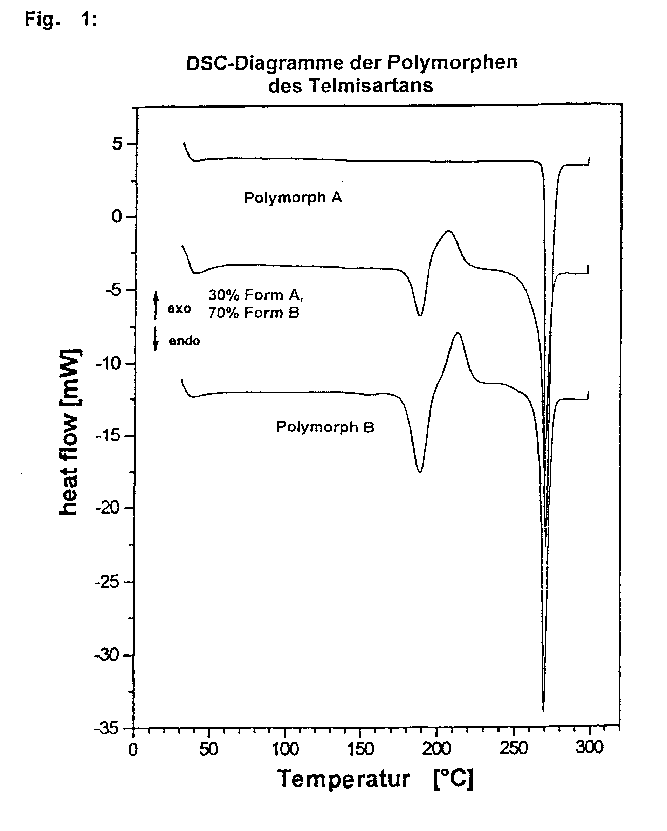

Die beiden Polymorphen Formen A und B des Telmisartans unterscheiden sich stark in ihrem Schmelzpunkt. Form B schmilzt bei 183+/-2°C (bestimmt über DSC), Form A bei 269+/-2°C (bestimmt über DSC). Nach dem Schmelzen kristallisiert die niedrig schmelzendere Form B des Telmisartans als Form A wieder aus. Dies findet seinen Niederschlag beispielsweise darin, daß dem mittels DSC bestimmten endothermen Maximum bei 183+/-2°C ein charakteristisches exothermes Maximum folgt, das die Kristallisation der Schmelze der Form B in die hochschmelzende Form A wiederspiegelt. Die mit einem Mettler DSC-20, TA8000 System erhaltenen DSC-Diagramme (DSC=Differential Scanning Calorimetry) sind in Figur 1 dargestellt.

Die Polymorphen A und B unterscheiden sich ebenfalls in ihrem IR-Spektrum. Aufgrund dieser Unterscheidung läßt sich die IR-Spektroskopie gegebenenfalls zur quantitativen Bestimmung des Verhältnisses der beiden Kristallmodifikationen nach der Trocknung im Endprodukt nutzen. Reines Polymorph A weist im IR-Spektrum eine charakteristische Bande bei 815cm⁻¹ auf. Beim Polymorph B ist diese Schwingung auf 830cm ⁻¹ verschoben. Da diese beiden charkteristischen Banden der Polymorphe A und B weit genug voneinander separiert sind, sind sie zur vorstehend genannten quantitativen Bestimmung des Verhältnisses der beiden Kristallmodifikationen besonders geeignet.

Die IR-spektroskopische Charakterisierung der beiden polymorphen Formen A und B erfolgte mit dem Nicolet FTIR Spectrometer Magna - IR 550 in KBr (2.5µmol pro 300 mg KBr; Nicolet software package OMNIC, version 1.20).

Die nachfolgenden Beispiele dienen der Illustration exemplarisch durchgeführter Reinigungs- und Kristallisationsverfahren zur Herstellung der polymorphen Form B des Telmisartan. Sie sind lediglich als mögliche, exemplarisch dargestellte Vorgehensweisen zu verstehen, ohne die Erfindung auf deren Inhalt zu beschränken.

### Beispiel 1

In einen 1200 I-Rührwerksapparat werden 205,6 kg Telmisartan-Umkristallisat (umkristallisiert aus Dimethylformamid oder Dimethylacetamid), 6,2 kg Aktivkohle, 205,6 l Wasser, 211,6 kg Ameisensäure (99-100%-ig) und 205,6 I Ethylacetat eingebracht. Es wird ca. 1 h bei 70 - 80°C gerührt und dann in einen weiteren 1200 I-Rührwerksapparat filtriert und mit einem Gemisch aus 82,2 I Ethylacetat und 9,2 kg Ameisensäure (99-100%-ig) nachgewaschen. Unter gleichzeitigem Eindosieren von 308 l Wasser destilliert man bei 80 - 100°C ca. 308 I Lösungsmittel ab. Danach wird auf 20 - 30°C abgekühlt und durch Eindosieren von 313 kg 25 %iger Ammoniaklösung gefällt. Das ausgefallene Produkt wird abgeschleudert, mit Wasser gewaschen und bei 120-125°C getrocknet.
Ausbeute : 200 kg Telmisartan (97,3 % d. Th.)

### Beispiel 2

In einen 1200 I-Rührwerksapparat werden 185 kg Telmisartan-Umkristallisat (umkristallisiert aus Dimethylformamid oder Dimethylacetamid), 5,6 kg Aktivkohle, 185 I Wasser, 190,4 kg Ameisensäure (99-100%-ig) und 185 I Tetrahydrofuran eingebracht. Es wird ca. 1 h bei 60 - 70°C gerührt und dann in einen weiteren 1200 I-Rührwerksapparat filtriert und mit einem Gemisch aus 74 l Tetrahydrofuran und 8,3 kg Ameisensäure (99-100%-ig) nachgewaschen. Unter gleichzeitigem Eindosieren von 278 l Wasser destilliert man bei 70 - 100°C ca. 278 l Lösungsmittel ab. Danach wird auf 20 - 30°C abgekühlt und durch Eindosieren von 281,5 kg 25 %iger Ammoniaklösung gefällt. Das ausgefallene Produkt wird abgeschleudert, mit Wasser gewaschen und bei
120-125°C getrocknet.
Ausbeute : 180 kg Telmisartan (97,3 % d. Th.)

### Beispiel 3

In einen 1200 I-Rührwerksapparat werden 185 kg Telmisartan-Umkristallisat (umkristallisiert aus Dimethylformamid oder Dimethylacetamid), 5,6 kg Aktivkohle, 1851 Wasser, 190,4 kg Ameisensäure (99-100%-ig) und 185 l Methylethylketon eingebracht. Es wird ca. 1 h bei 60 - 70°C gerührt und dann in einen weiteren 1200 I-Rührwerksapparat filtriert und mit einem Gemisch aus 74 l Methylethylketon und 8,3 kg Ameisensäure (99-100%-ig) nachgewaschen. Unter gleichzeitigem Eindosieren von 278 l Wasser destilliert man bei 80 -100°C ca. 278 l Lösungsmittel ab. Danach wird auf 20 -30°C abgekühlt und durch Eindosieren von 281,5 kg 25 %iger Ammoniaklösung gefällt. Das ausgefallene Produkt wird abgeschleudert, mit Wasser gewaschen und bei 120-125°C getrocknet.
Ausbeute : 178 kg Telmisartan (96,2 % d. Th.)

### Vergleichsbeispiel

In einen 1200 I-Rührwerksapparat werden 150 kg Telmisartan (umkristallisiert aus Dimethylformamid oder Dimethylacetamid), 7,5 kg Aktivkohle, 750 l Ethanol und 30 kg 25 %ige wäßrige Ammoniaklösung eingebracht. Es wird ca. 1 h gerührt und dann in einen weiteren 1200 I-Rührwerksapparat filtriert und mit 150 l Ethanol nachgewaschen. Man erhitzt auf 70 - 80°C, gibt 35 kg Eisessig zu und rührt noch 1,5 - 2 h bei 75 - 80 °C. Danach wird auf 0 - 10 °C abgekühlt und nochmals 2 h gerührt. Das ausgefallene Produkt wird abgeschleudert, mit 300 I Ethanol und mit 300 I Wasser gewaschen und bei 70 - 90°C getrocknet.
Ausbeute : 135 kg Telmisartan (90 % d. Th.) reine Form A

Telmisartan fällt im erfindungsgemäßen Herstellungsprozess aufgrund der teilweisen Umwandlung der polymorphen Form B in die polymorphe Form A während des Trocknungsvorgangs als Reinsubstanz in einem Gemisch zweier polymorpher Formen an. Dies hat allerdings keinen Einfluß auf die Eigenschaften des Arzneimittels, denn beispielsweise im Rahmen der Herstellung von Telmisartan-Tabletten wird das Gemisch der Polymorphen Formen A und B in 0.1 N NaOH-Lösung gelöst und über Sprühtrocknung in ein homogenes und vollständig amorphes Granulat überführt, welches anschließend den weiteren Tablettenfertigungsschritten zugeführt wird. Für weitergehende, detailliertere Angaben bezüglich der Verwendung der erfindungsgemäßen Produkte zur Herstellung eines Arzneimittels sei auf die EP 502314 B1 verwiesen, auf die an dieser Stelle inhaltlich Bezug genommen wird.

## Patentansprüche

1. Polymorphe Kristallmodifikation B (Form B) des Telmisartans (Formel I), 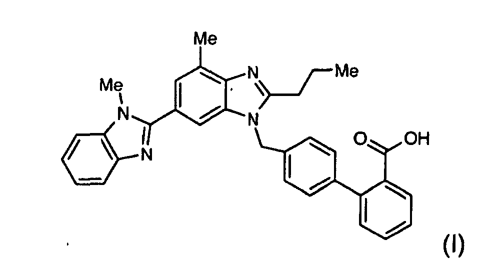 **gekennzeichnet durch** ein bei der thermischen Analyse mittels DSC auftretendes endothermes Maximum bei 183±2°C.

2. Telmisartan, **gekennzeichnet durch** einen Gehalt an Form B gemäß Anspruch 1.

3. Verfahren zur Herstellung von Telmisartan gemäß einem der Ansprüche 1 oder 2, **dadurch gekennzeichnet, daß**
a) Telmisartan in einem Lösemittelgemisch bestehend aus Wasser, Ameisensäure und einem damit mischbaren organischen Lösemittel, ausgewählt aus organischen Carbonsäureestern, Ketonen oder Ethern aufgenommen, erwärmt und die erhaltene Lösung anschließend filtriert wird,
b) das organische Lösemittel - gegebenenfalls unter gleichzeitiger Zudosierung von Wasser - abdestilliert wird,
c) das Telmisartan Form B aus der verbleibenden Lösung durch Zugabe einer Base gefällt wird und
d) das ausgefallene Produkt abgeschleudert, gewaschen und getrocknet wird.

4. Verfahren gemäß Anspruch 3, **dadurch gekennzeichnet, daß** als organisches Lösemittel Aceton, Methylethylketon, Methylacetat, Ethylacetat, Ethylformiat, Ethylenglykoldimethylether oder Tetrahydrofuran eingesetzt werden.

5. Verfahren gemäß Anspruch 3 oder 4, **dadurch gekennzeichnet, daß** als organisches Lösemittel Aceton, Methylethylketon, Methylacetat, Ethylacetat oder Tetrahydrofuran eingesetzt werden.

6. Verfahren gemäß Anspruch 3, 4 oder 5, **dadurch gekennzeichnet, daß** als organisches Lösemittel Ethylacetat eingesetzt wird.

7. Verfahren gemäß Anspruch 3, 4, 5 oder 6, **dadurch gekennzeichnet, daß** als Base Ammoniak verwendet wird.

8. Verwendung von Telmisartan gemäß Anspruch 1 oder 2 zur Herstellung eines Arzneimittels.

## Claims

1. Polymorphic crystal modification B (form B) of telmisartan (formula I), 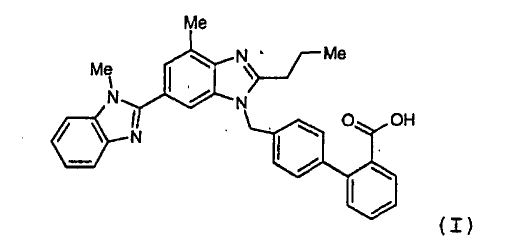 **characterised by** an endothermic maximum at 183±2°C which occurs during thermal analysis using DSC.

2. Telmisartan, **characterised in that** it contains form B according to claim 1.

3. Process for preparing telmisartan according to one of claims 1 or 2, **characterised in that**
a) telmisartan is taken up in a mixture of solvents consisting of water, formic acid and an organic solvent miscible therewith, selected from among the organic caboxylic acid esters, ketones or ethers, heated, and the resulting solution is then filtered,
b) the organic solvent is distilled off, optionally while water is simultaneously added in metered amounts,
c) the telmisartan form B is precipitated out of the remaining solution by the addition of a base and
d) the product precipitated is centrifuged, washed and dried.

4. Process according to claim 3, **characterised in that** acetone, methylethylketone, methyl acetate, ethyl acetate, ethyl formate, ethyleneglycol dimethylether or tetrahydrofuran is used as the organic solvent.

5. Process according to claim 3 or 4, **characterised in that** acetone, methylethylketone, methyl acetate, ethyl acetate or tetrahydrofuran is used as the organic solvent.

6. Process according to claim 3, 4 or 5, **characterised in that** ethyl acetate is used as the organic solvent.

7. Process according to claim 3, 4, 5 or 6, **characterised in that** ammonia is used as the base.

8. Use of telmisartan according to claim 1 or 2 for preparing a medicament.

## Revendications

1. Modification cristalline polymorphe B (forme B) du telmisartan (formule I) 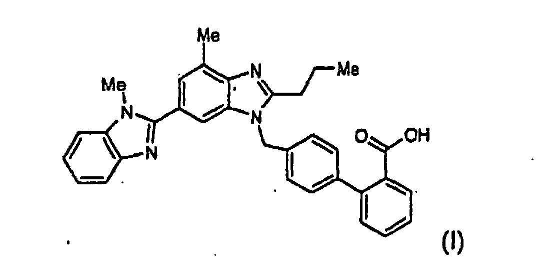 **caractérisée par** l'apparition d'un maximum endothermique à 183 ± 2°C lors de l'analyse thermique par DSC.

2. Telmisartan **caractérisé par** une teneur en la forme B selon la revendication 1.

3. Procédé de préparation du telmisartan selon l'une des revendications 1 et 2 **caractérisé en ce que**
a) le telmisartan est introduit dans un mélange de solvants consistant en eau, acide formique et un solvant organique miscible aux précédents, choisi parmi les esters d'acides carboxyliques, cétones ou éthers organiques, chauffé et la solution obtenue est ensuite filtrée,
b) le solvant organique est retiré par distillation, éventuellement avec addition simultanée d'eau,
c) la forme B du telmisartan est précipitée dans la solution restante par addition d'une base, et
d) le produit précipité est centrifugé, lavé et séché.

4. Procédé selon la revendication 3 **caractérisé en ce que** l'acétone, la méthyléthylcétone, l'acétate de méthyle, l'acétate d'éthyle, le formiate d'éthyle, le diméthyléther d'éthylèneglycol ou le tétrahydrofurane est utilisé comme solvant organique.

5. Procédé selon la revendication 3 ou 4 **caractérisé en ce que** l'acétone, la méthyléthylcétone, l'acétate de méthyle, l'acétate d'éthyle ou le tétrahydrofurane est utilisé comme solvant organique.

6. Procédé selon la revendication 3, 4 ou 5 **caractérisé en ce que** l'acétate d'éthyle est utilisé comme solvant organique.

7. Procédé selon la revendication 3, 4, 5 ou 6 **caractérisé en ce que** l'ammoniac est utilisé comme base.

8. Utilisation du telmisartan selon la revendication 1 ou 2 pour la préparation d'un médicament.
